# EUROPEAN PATENT SPECIFICATION

(11) **EP 0 773 769 B1**
(45) Date of publication and mention of the grant of the patent: **02.11.2000**
(21) Application number: 95926295.7
(22) Date of filing: 14.07.1995
(51) Int. Cl.: A61F 13/15

(54) **ABSORBENT ARTICLE WITH ELASTICIZED SIDE PANELS CONNECTED BY A BRIDGE MEMBER**
ABSORBIERENDER ARTIKEL MIT MIT ELASTIFIZIERTEN SEITENTEILEN VERBUNDEN DURCH EIN BRÜCKENGLIED
ARTICLE ABSORBANT AVEC DES PANNEAUX LATERAUX ELASTIQUES, RELIES PAR UN ELEMENT DE LIAISON

(30) Priority: 03.08.1994 US 286491
(43) Date of publication of application: 21.05.1997
(73) Proprietor: KIMBERLY-CLARK WORLDWIDE, INC., Neenah, Wisconsin 54956 (US)
(72) Inventor: FRIES, Merlin, Donald, Combined Locks, WI 54113 (US); HUNTOON, Andrew, Edsel, Appleton, WI 54915 (US); O'ROURKE, Kathleen, Ann, Neenah, WI 54956 (US); PETERSON, Dale, Arthur, Neenah, WI 54956 (US); STROHBEEN, Irene, Beatrice, Menasha, WI 54952 (US)
(74) Representative: Diehl, Hermann, Dr. Dipl.-Phys.
(86) International application number: US9508904
(87) International publication number: WO9603953

(56) References cited:
- EP-A- 0 242 766
- EP-A- 0 443 082
- WO-A-93/09746
- WO-A-93/24085
- WO-A-94/28844
- FR-A- 2 680 316
- GB-A- 2 244 422
- US-A- 5 019 066
- US-A- 5 019 073

## Description

### Field of the Invention

The present invention relates to an elasticized article. More particularly, the invention relates to an absorbent article having elasticized side panels connected to a bridge member to provide a composite bridge member which can be more efficiently assembled into the article, and can provide improved performance attributes.

### Background of the Invention

Conventional absorbent articles, such as disposable diapers, have been constructed with elasticized waistbands. Particular article designs have incorporated a stretchable outer cover composed of an elastomeric web material, such as a stretch bonded laminate which includes a layer of nonwoven fabric. Other conventional designs have included elastomeric side panel members connected to the lateral side edges of a polymer film material. For example, see U.S. Patent 4,861,652 to Lippert et al.; U.S. Patent 4,701,170 issued October 20, 1987, to Wilson et al.; and U.S. Patent 5,019,073 issued May 28, 1991, to Roessler et al.

Conventional absorbent articles, such as those described above, have not been completely satisfactory when subjected to high loadings of liquids, such as urine. As a result, there has remained a continuing need for absorbent articles that can provide improved comfort and fit, and increased resistance to leakage.

FR-A-2 680 316 discloses a diaper comprising an external sheet impervious to liquids, an absorbing mattress, and an internal sheet permeable to liquids. Two transverse liquid-repellent bands are fixed to the interal sheet in the front part and in the back part so as to form liquid-repellent waist pockets, said bands having dimensions in the transverse direction which are larger than the width of the external impervious sheet and carrying adhesive fastening devices.

### Brief Description of the Invention

The present invention is directed to an absorbent article (10) having a front waistband section (12), a rear waistband section (14) and an intermediate section (16) which interconnects said front and rear waistband sections, said article comprising:
a backsheet layer (30) having a laterally extending width and a longitudinally extending length;
a liquid permeable topsheet layer (28) superposed on said backsheet layer (30), said topsheet layer (28) having a laterally extending width and a longitudinally extending length;
an absorbent body (32) located between said backsheet layer (30) and said topsheet layer (28);
a bridge member (40) which is provided separate from said backsheet (30) and topsheet (28) layers, at least a portion of said bridge member (40) arranged in an overlapping relation with a longitudinal end section of said absorbent body (32) and connected to at least one of said backsheet (30) and topsheet (28) layers;
   and
   fastening means (36) for securing said article (10) on a wearer,
said article characterised in that it further comprises elasticized side panels (56, 58) which are provided separate from said bridge member and are connected to said bridge member (40) at each laterally opposed end section (84, 86) of said bridge member (40);
said side panels (56, 58) are constructed to be elastically stretchable at least along a lateral cross-direction of said article (10);
said bridge member (40) extends laterally to interconnect between said elasticized side panels (56, 58);
said fastening means (36) is connected to a laterally distal end region of at least one of said side panels (56, 58);
said bridge member (40) is configured to form an integrated subassembly with said side panels, said subassembly maintaining a desired lateral separation and alignment between said separately provided side panels (56, 58) when said sub-assembly is positioned in said article during manufacturing.

The absorbent article has a front waistband section, a rear waistband section and an intermediate section which interconnects the front and rear waistband sections.

The article includes a backsheet layer having a laterally extending width and a longitudinally extending length. A liquid permeable topsheet layer is superposed on the backsheet layer, and the topsheet layer has a laterally extending width and a longitudinally extending length. An absorbent body is located between the backsheet layer and the topsheet layer, and an elasticized side panel is connected to the article at each laterally opposed end region of at least one of the front and rear waistband sections. The side panels are constructed to be elastically stretchable at least along a lateral, cross-direction of the article. A bridge member, which is separate from the backsheet and topsheet layers, extends laterally to interconnect between the elasticized side panels. The bridge member is arranged in an overlapping relation with a longitudinal end section of the absorbent body, and is connected to at least one of the backsheet and topsheet layers. A fastening means for securing the article on a wearer is connected to a laterally distal end region of at least one of the side panels.

### Brief Description of the Drawings

The invention will be more fully understood and further advantages will become apparent when reference is made to the following detailed description and accompanying drawings in which:
Fig. 1 representatively shows a partially cut-away, top view of an article of the invention having the bridge member positioned onto a major, facing surface of a backsheet layer of the article;
Fig. 2 representatively shows a schematic, lateral cross-sectional view taken through Section 2-2 of Fig. 1;
Fig. 3 representatively shows a schematic, longitudinal cross-sectional view taken through Section 3-3 of Fig. 1
Fig. 4 representatively shows a partially cut-away, top view of an article of the invention having the bridge member located generally adjacent an outwardly facing surface of the topsheet layer in an arrangement which overlaps a longitudinal end section of an absorbent body of the article;
Fig. 5 representatively shows a schematic, lateral cross-sectional view taken along Section 5-5 of Fig. 4;
Fig. 6 representatively shows a schematic longitudinal cross-sectional view taken through Section 6-6 of Fig. 4;
Fig. 7 representatively shows a partially cut-away, top view of an article of the invention having the bridge member located generally adjacent a bodyside surface of the topsheet layer of the article;
Fig. 8 representatively shows a schematic, lateral cross-sectional view taken along Section 8-8 of Fig. 7;
Fig. 9 representatively shows a schematic, longitudinal cross-sectional view taken through Section 9-9 of Fig. 7;
Fig. 10 representatively shows a partially cut-away, top view of an article of the invention having elasticized containment flaps and having the bridge member overlying the flaps;
Fig. 11 representatively shows a schematic, lateral cross-sectional view taken along Section A-A of Fig. 7;
Fig. 12 representatively shows a schematic, longitudinal cross-sectional view taken through Section B-B of Fig. 7.

### Detailed Description of the Invention

The structures of the present invention will be described herein in relationship to their use in disposable absorbent articles, but it should be understood that potential uses of the structures of the present invention need not be limited to disposable absorbent articles. As used herein, the term "disposable absorbent article" refers to articles which absorb and contain body exudates and are intended to be discarded after a limited period of use. The articles are not intended to be laundered or otherwise restored for reuse. The articles can be placed against or in proximity to the body of the wearer to absorb and contain various exudates discharged from the body. While the present description will particularly be made in the context of a diaper article, it should be understood that the present invention is also applicable to other disposable personal care absorbent articles, such as adult incontinence garments, sanitary napkins, children's training pants and the like.

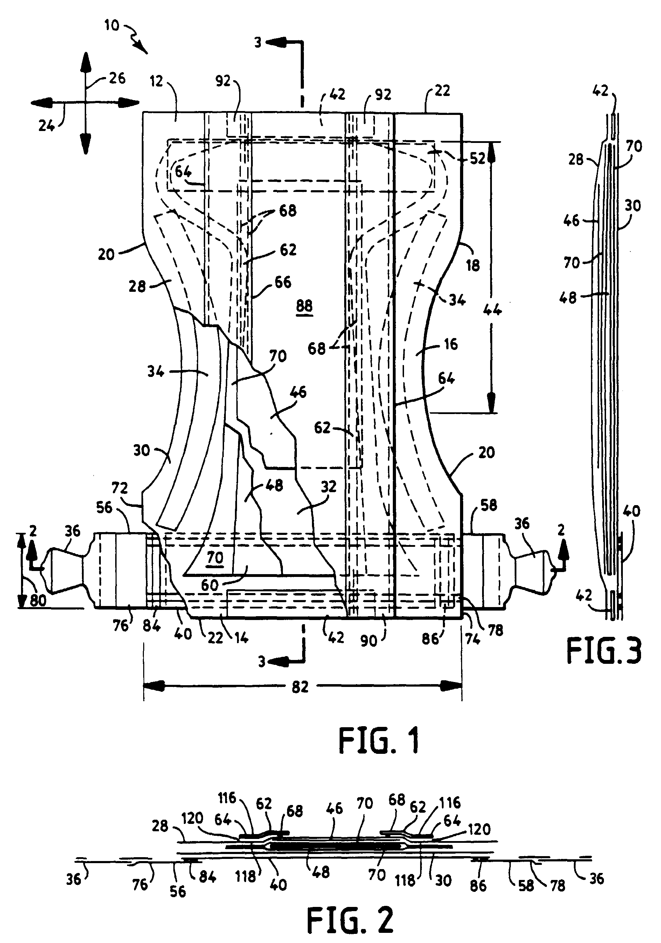

With reference to Figs. 1, 2 and 3, an absorbent article, such as diaper 10, has a cross-wise, lateral dimension 24 and a length-wise, longitudinal dimension 26. The representative diaper 10, has a front waistband section 12, a rear or back waistband section 14, and an intermediate section 16 which interconnects the front and rear waistband sections. The article includes a backsheet layer 30 having a laterally extending width and a longitudinally extending length. A porous, liquid permeable topsheet layer 28 has a laterally extending width and a longitudinally extending length, and is superposed on the backsheet layer 30. An absorbent structure, such as absorbent body 32 is located between the backsheet layer 30 and the topsheet layer 28. An elasticized side panel 56 is connected to the article at each laterally opposed end region 72 and 74 of at least one of the front and rear waistband sections. The side panels 56 are constructed to be elastically stretchable at least along a laterally extending cross-direction 24 of the article. A bridge member 40 which is separate from the backsheet and topsheet layers extends laterally along cross-direction 24 to interconnect between the elasticized side panels 56 and 58, and is constructed to maintain a desired lateral separation and alignment between the side panels. At least a portion of the bridge member 40 is arranged in an overlapping relation with a longitudinal end section 60 of the absorbent body 32, and is connected to at least one of the backsheet and topsheet layers. A fastening means, such as provided by tape tab fasteners 36, is provided for securing the article on a wearer. The fastening means connect to a laterally distal end region 76 and/or 78 of at least one of the side panels 56 and 58. In the illustrated embodiment, for example, a first tape tab fastener 36 is connected to the distal end region 76 of side panel 56, and a second tape tab fastener is connected to the distal end region 78 of elasticized side panel 58.

Fig. 1 is a representative plan view of diaper 10 of the present invention in its flat-out, uncontracted state (i.e., with all elastic induced gathering and contraction removed). Portions of the structure are partially cut away to more clearly show the interior construction of diaper 10, and the surface of the diaper which contacts the wearer is facing the viewer. In the shown embodiment, diaper 10 has a front waistband region 12, a back waistband region 14, an intermediate crotch region 16 which interconnects the front and rear waistband regions. The outer edges of the diaper define a periphery 18 in which the longitudinally extending side edge margins are designated 20 and the laterally extending end edge margins are designated 22. The side edges define leg openings for the diaper, and optionally, are curvilinear and contoured. The end edges are shown as straight, but optionally, may be curvilinear. The diaper additionally has a transversely extending, lateral width dimension 24 and a longitudinal, length dimension 26.

Diaper 10 typically includes a porous, liquid permeable topsheet 28; a substantially liquid impermeable backsheet 30; an absorbent structure 32, positioned between the topsheet and backsheet; a surge management portion 46; and elastic members, such as leg elastics 34 and waist elastics 42. The surge management portion is positioned in liquid communication with the absorbent structure, and the absorbent structure includes a retention portion 48. Topsheet 28, backsheet 30, absorbent structure 32, surge management portion 46 and the elastic members 34 and 42 may be assembled in a variety of well-known diaper configurations. In addition, the diaper can include a system of containment flaps 62.

As representatively shown, topsheet 28 and backsheet 30 may be generally coextensive, and may have length and width dimensions which are generally larger than the corresponding dimensions of absorbent structure 32. Topsheet 28 is associated with and superimposed on backsheet 30, thereby defining the periphery 18 of diaper 10. The periphery delimits the outer perimeter of the diaper 10, and in the illustrated embodiment, comprises laterally marginal end edges 22, and contoured longitudinally extending marginal side edges 20. The diaper 10 has front and back waistband regions 12 and 14, respectively, extending from the laterally extending end edges 22 of diaper periphery 18 toward the transverse center line of the diaper along a distance of from about 2 percent to about 10 percent of the overall length of diaper 10. The waistband regions comprise those upper portions of diaper 10, which when worn, wholly or partially cover or encircle the waist or mid-lower torso of the wearer. The intermediate, crotch region 16 lies between and interconnects waistband regions 12 and 14, and comprises that portion of diaper 10 which, when worn, is positioned between the legs of the wearer and covers the lower torso of the wearer. Thus, the crotch region 16 is an area where repeated fluid surge typically occur in the diaper or other disposable absorbent article.

Topsheet 28 presents a body-facing surface which is compliant, soft-feeling, and non-irritating to the wearer's skin. Further, topsheet 28 can be less hydrophilic than retention portion 48, and is sufficiently porous to be liquid permeable, permitting liquid to penetrate through its thickness. The topsheet layer has marginal side regions 94 and 96, and has marginal end regions 108.

A suitable topsheet 28 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. Topsheet 28 is typically employed to help isolate the wearer's skin from liquids held in absorbent structure 32. Various woven and nonwoven fabrics can be used for topsheet 28. For example, the topsheet may be composed of a meltblown or spunbonded web of polyolefin fibers. The topsheet may also be a bonded-carded-web composed of natural and/or synthetic fibers.

For the purposes of the present description, the term "nonwoven web" means a web of material which is formed without the aid of a textile weaving or knitting process. The term "fabrics" is used to refer to all of the woven, knitted and nonwoven fibrous webs.

The topsheet fabrics may be composed of a substantially hydrophobic and substantially nonwettable material, and the hydrophobic material may optionally be treated with a surfactant or otherwise processed to impart a desired level of wettability and hydrophilicity. In a particular embodiment of the invention, topsheet 28 can be a nonwoven, spunbond polypropylene fabric composed of about 2.8-3.2 denier fibers formed into a web having a basis weight of about 22 gsm and density of about 0.06 gm/cc. The fabric can be surface treated with a selected amount of surfactant, such as about 0.28% Triton X-102 surfactant. The surfactant can be applied by any conventional means, such as spraying, printing, brush coating or the like.

The surfactant material, such as a conventional wetting agent, can be applied to a medial section of the topsheet layer 28 to provide a greater wettability of the medial section, as compared to a remainder of the topsheet layer 28. In particular configurations, the cross-directional width of the medial section can be substantially equal to or less than the cross-directional width of the surge management portion 46. In alternative configurations, the medial section width can be substantially equal to or less than a cross-directional spacing between a pair of adhesive strips employed to secure the containment flaps 62 onto topsheet 28 and to form a leak resistant barrier seal onto the backsheet 30.

The surfactant-treated medial section can be approximately centered with respect to the longitudinal centerline of the diaper, and can extend along substantially the entire length of the topsheet layer. Alternatively, the surfactant treated medial section can be constructed to extend along only a predetermined portion of the topsheet length.

The various configurations of the invention can include elasticized containment flaps 62. The shown configurations, for example, include two containment flaps 62 which are connected to the bodyside surface of topsheet layer 28. Suitable constructions and arrangements for containment flaps 62 are described, for example, in U.S. Patent 4,704,116 issued November 3, 1987, to K. Enloe.

Other configurations of the containment flaps 62 are described in U.S. Patent Application Serial No. 208,816 of R. Everett et al., filed March 4, 1994 and entitled ABSORBENT ARTICLE HAVING AN IMPROVED SURGE MANAGEMENT (Attorney docket No. 11,375).

Such containment flaps can be attached to topsheet layer 28 along length-wise extending fixed regions, such as fixed edges 64, of the flaps. A movable edge 66 of each containment flap includes a flap elastic member 68 comprising one or more individual strands of elastomeric material. For example, a plurality of elastic strands may be configured in a spatially separated, generally parallel arrangement, and a suitable elastic strand can, for example, be composed of a 470 decitex Lycra elastomer. Elastic member 68 is connected to the movable edge of the containment flap in an elastically contractible condition such that the contraction of the elastic components thereof gathers and shortens the edge of the containment flap. As a result, the movable edge of each containment flap tends to position itself in a spaced relation away from the bodyside surfaces of topsheet 28 and/or surge management portion 46 toward a generally upright and approximately perpendicular configuration, especially in the crotch section of the diaper. In the shown embodiment, for example, the moveable edge of the barrier flap is connected to the flap elastics by partially doubling the flap material back upon itself by a limited amount which is sufficient to enclose flap elastics 68.

At least a pair of containment or barrier flaps 62 can be connected to laterally opposed, longitudinally extending regions of topsheet layer 28, and the connected topsheet regions are located generally adjacent to laterally opposed side edge regions of the medial section of topsheet layer 28. The connected topsheet regions are also located substantially laterally inboard of the elasticized side margins of the diaper article 10.

The containment flaps may, for example, be constructed of a fibrous material which is similar to the material comprising topsheet 28, or similar to the material comprising surge management portion 46. Other conventional materials, such as polymer films, may also be employed. In other aspects of the invention, barrier flaps 62 are constructed of a material which is permeable to gas, such as ambient air. Alternative configurations of the invention can include barrier flaps which are constructed of a material which is resistant to a passage of aqueous liquid, such as urine, therethrough. For example, barrier flaps 62 may be constructed of a spunbond-meltblown-spunbond (SMS) laminate material. In the illustrated embodiment, for example, the barrier flaps can be constructed of a SMS material having a basis weight of about 0.85 osy (about 28 gsm). The spunbond layers are composed of polypropylene fibers, and the meltblown layer is composed of meltblown polypropylene fibers.

In the various configurations of the invention, such as where the barrier flaps 62 are configured to be permeable to gas while having a resistance and limited permeability to aqueous liquid, the liquid resistent material can have a construction which is capable of supporting a hydrohead of at least about 45 cm of water substantially without leakage therethrough. A suitable technique for determining the resistance of a material to liquid penetration is Federal Test Method Standard FTMS 191 Method 5514, dated 31 December 1968.

With reference to Figs. 2, each of the barrier flaps 62 can include a laterally extending base section 116 thereof with at least a portion of the base section attached to the topsheet layer 28 at a topsheet securement section 118, which is located laterally outboard of the retention portion 48 and laterally outboard of the surge management portion 46. The topsheet securement section 118 of topsheet layer 28 is operably sealed to the backsheet layer 30 to substantially prevent or block a leakage of liquid through the securement section 118. The securement section can be constructed by employing various conventional techniques, such as adhesive bonding, thermal bonding, sonic bonding, stitching, stapling, or the like. The illustrated embodiment, for example, is configured with the topsheet securement section 118 sealed to the backsheet layer 30 with a substantially continuous adhesive bead or strip 120 composed of a pressure-sensitive, hot melt adhesive.

The adhesive strip can advantageously provide a barrier bead which extends generally length-wise of the diaper, and at least a portion of the barrier bead is located in the diaper crotch region 16. The crotch portion of the barrier bead can be constructed to operably bond and substantially seal the corresponding portion of the securement section 118 of topsheet layer 28 to both the barrier flap base section 116 and the backsheet layer 30 by effectively "bleeding" through the topsheet layer 28 to make operable contact with the backsheet layer. When a substantially continuous seal is provided, liquid can be more effectively contained between the two flaps 62. The representative adhesive strip 120 is wiped or otherwise applied onto the appointed section of the flap base 116 at a position which interposes the strip between the terminal side edge of tissue wrap 70 and the leg elastic carrier sheet. Additional pressure can be applied to the adhesive strip area to help assure a desired seal.

At least a portion of the base section can also be attached to the topsheet layer 28 along a topsheet seam section located along the fixed edge 64 of the containment flap. A suitable connecting means, such as a substantially continuous adhesive bead, operably secures the fixed barrier flap edge to the topsheet seam section.

Backsheet 30 may be composed of a liquid permeable material, but preferably comprises a material which is configured to be substantially impermeable to liquids. For example, a typical backsheet can be manufactured from a thin plastic film, or other flexible liquid-impermeable material. As used in the present specification, the term "flexible" refers to materials which are compliant and which will readily conform to the general shape and contours of the wearer's body. Backsheet 30 can help prevent the exudates contained in absorbent structure 32 from wetting articles such as bedsheets and overgarments which contact diaper 10.

In particular embodiments of the invention, backsheet 30 is a polyethylene film having a thickness of from about 0.012 millimeters (0.5 mil) to about 0.051 millimeters (2.0 mils). In the shown embodiment, the backsheet is a film having a thickness of about 0.032 mm (about 1.25 mil). Alternative constructions of the backsheet may comprise a woven or non-woven fibrous web layer which has been totally or partially constructed or treated to impart the desired levels of liquid impermeability to selected regions that are adjacent or proximate the absorbent body. Backsheet 30 typically provides the outer cover of the article. Optionally, however, the article may comprise a separate outer cover member which is in addition to the backsheet.

Backsheet 30 may optionally be composed of a micro-porous, "breathable" material which permits vapors to escape from absorbent structure 32 while still preventing liquid exudates from passing through the backsheet. For example, the breathable backsheet may be composed of a microporous polymer film or a nonwoven fabric which has been coated or otherwise treated to impart a desired level of liquid impermeability. For example, a suitable microporous film is a PMP-1 material, which is available from Mitsui Toatsu Chemicals, Inc., a company having offices in Tokyo, Japan; or an XKO-8044 polyolefin film available from 3M Company of Minneapolis, Minnesota. The backsheet can also be embossed or otherwise be provided with a matte finish to exhibit a more aesthetically pleasing appearance.

The size of backsheet 30 is typically determined by the size of absorbent structure 32 and the exact diaper design selected. Backsheet 30, for example, may have a generally T-shape, a generally I-shape or a modified hourglass shape, and may extend beyond the terminal edges of absorbent structure 32 by a selected distance, such as a distance within the range of about 1.3 centimeters to 2.5 centimeters (about 0.5 to 1.0 inch), to provide side margins.

Topsheet 28 and backsheet 30 are connected or otherwise associated together in an operable manner. As used therein, the term "associated" encompasses configurations in which topsheet 28 is directly joined to backsheet 30 by affixing topsheet 28 directly to backsheet 30, and configurations wherein topsheet 28 is joined to backsheet 30 by affixing topsheet 28 to intermediate members which in turn are affixed to backsheet 30. Topsheet 28 and backsheet 30 can be affixed directly to each other in the diaper periphery 18 by attachment means (not shown) such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet 28 to backsheet 30.

It should be readily appreciated that the above-described attachment means may also be employed to interconnect and assemble together the various other component parts of the article described herein.

In the representatively shown embodiment of the invention, the topsheet layer 28 is disposed and secured in facing relation with the backsheet layer 30 to retain and hold the retention portion 48 and the surge management 45 between the backsheet layer and the topsheet layer. The marginal side regions of topsheet layer 28 are operably connected to corresponding marginal side regions of the backsheet layer 30. Each of the attached marginal side regions of the topsheet and backsheet layers is located laterally outboard of its corresponding, associated side edge region of the surge management portion 45. In particular configurations of the invention, the attached marginal regions of topsheet 28 can include marginal end regions. The attached marginal end regions are located longitudinally outboard of the end edge regions of the retention portion 48 and/or surge management portion 46. Similarly, the attached marginal regions of backsheet 30 can include attached marginal end regions, which can be located longitudinally outboard of the end edge regions of the retention portion and/or surge management portion.

Elastic members 34 are disposed adjacent the periphery 18 of diaper 10 along each of the longitudinal side edges 20. The leg elastic members 34 can be connected to either or both of the topsheet and backsheet layers to provide elasticized side margins of the diaper article, and can be arranged to draw and hold diaper 10 against the legs of the wearer. Waist elastic members 42 may also be disposed adjacent either or both of the end edges of diaper 10 to provide elasticized waistbands.

Elastic members 34 and 42 are secured to diaper 10 in an elastically contractible condition so that in a normal under strain configuration, the elastic members effectively contract against diaper 10. The elastic members can be secured in an elastically contractible condition in at least two ways, for example, the elastic members may be stretched and secured while diaper 10 is in an uncontracted condition. Alternatively, diaper 10 may be contracted, for example, by pleating, and the elastic members secured and connected to diaper 10 while the elastic members are in their unrelaxed or unstretched condition. Still other means, such as heat-shrink elastic material, may be used to gather the garment.

In the embodiment illustrated in Fig. 1, leg elastic members 34 extend essentially along the complete length of crotch region 16 of diaper 10. Alternatively, elastic members 34 may extend the entire length of diaper 10, or any other length suitable providing the arrangement of elastically contractible lines desired for the particular diaper design.

Elastic members 34 and 42 may have any of a multitude of configurations. For example, the width of the individual elastic members 34 may be varied from 0.25 millimeters (0.01 inches) to 25 millimeters (1.0 inches) or more. The elastic members may comprise a single strand of elastic material, or may comprise several parallel or non-parallel strands of elastic material, or may be applied in a rectilinear or curvilinear arrangement. Where the strands are non-parallel, two or more of the strands may intersect or otherwise interconnect within the elastic member. The elastic members may be affixed to the diaper in any of several ways which are known in the art. For example, the elastic members may be ultrasonically bonded, heat and pressure sealed using a variety of bonding patterns, or adhesively bonded to diaper 10 with sprayed or swirled patterns of hotmelt or other type of adhesive.

In the illustrated embodiments of the invention, for example, leg elastic members 34 may comprise a carrier sheet to which are attached a grouped set of elastics composed of a plurality of individual elastic strands 39. The elastic strands may intersect or be interconnected, or be entirely separated from one another. The shown carrier sheet may, for example, comprise a 0.002 cm thick film of unembossed polypropylene material. The shown elastic strands can, for example, be composed of LYCRA elastomer available from DuPont, a business having offices in Wilmington, Delaware. Each elastic strand is typically within the range of about 620-1050 decitex (dtx), and preferably, is about 940 dtx in an embodiment of the invention wherein three strands are employed for each elasticized legband. In addition, leg elastics 34 may be generally straight or optionally curved. For example, the curved elastics can be inwardly bowed toward the longitudinal centerline of the diaper with the innermost point (or apex, relative to the cross-direction of the article) of the set of curved elastic strands positioned approximately 1.905 - 3.81 cm (0.75 - 1.5 inches) inward from the outer most edge of the set of elastic strands. In particular arrangements, the curvature of the elastics may not be configured or positioned symmetrically relative to the lateral centerline of the diaper. As representatively shown in Fig. 1, the curved elastics may have an inwardly bowed and outwardly bowed, reflex-type of curvature, and the length-wise center of the elastics may be offset by a selected distance within the range of about 0-8 cm toward either the front or rear waistband of the diaper to provide desired fit and appearance. In particular embodiments of the invention, the innermost point (apex) of the set of curved elastics can be offset about 0-12 cm towards the front or rear waistband of the diaper, and the outwardly bowed reflexed-portion can be positioned toward the diaper front waistband.

An absorbent body, such as absorbent structure 32, is positioned between topsheet 28 and backsheet 30 to form diaper 10. The absorbent body has a construction which is generally compressible, conformable, non-irritating to the wearer's skin, and capable of absorbing and retaining liquid body exudates. It should be understood that, for purposes of this invention, the absorbent structure may comprise a single, integral piece of material, or alternatively, may comprise a plurality of individual separate pieces of material which are operably assembled together. Where the absorbent structure comprises a single, substantially integral piece of material, the material could include the desired structural features formed into selected spatial regions thereof. Where the absorbent structure comprises multiple pieces, the pieces may be configured as discrete layers or as other nonlayered shapes and configurations. Furthermore, the individual pieces may be coextensive or non-coextensive, depending upon the requirements of the product. It is preferred, however, that each of the individual pieces be arranged in an operable, intimate contact along at least a portion of its boundary with at least one other adjacent piece of the absorbent structure. Preferably, each piece is connected to an adjacent portion of the absorbent structure by a suitable bonding and/or fiber entanglement mechanism, such as ultrasonic or adhesive bonding, or mechanical or hydraulic needling.

In the embodiment representatively shown in Fig. 1, absorbent structure 32 includes a liquid-acquisition, target zone 44, and has a contoured, curvilinear periphery, particularly along its side edges. The two generally mirror-image, inwardly bowed, lateral edges provide for a narrower intermediate section suitable for positioning in the crotch of the wearer. In the shown absorbent structure 32, a front section thereof includes two transversely spaced ear regions and a central region. Target zone 44 encompasses the area where repeated liquid surges typically occur in absorbent structure 32. The particular location where liquid is discharged, such as during urination, can vary depending on the age and gender of the wearer. For example, male infants tend to urinate further toward the front end of the diaper. The female target zone is located closer to the center of the crotch. As a result, the shape and relative longitudinal placement of surge management portion 46 can be selected to best correspond with the actual target zone of either or both categories of wearers. Generally stated, the target zone is a section of absorbent structure 32 which is located in the front 60% of the length of the absorbent structure. With reference to the percentage of the total length of absorbent structure 32 measured into the absorbent structure from the front waistband edge thereof, the target zone may preferably comprise a region which begins at a line positioned approximately 10% of the absorbent structure length away from the front waistband edge and ends at approximately 60% of the absorbent structure length away from the front waistband edge.

Ear regions of the absorbent structure comprise portions which generally extend inwardly from the outermost lateral side edges of the absorbent structure toward its longitudinal center line. Thus, when the diaper is worn, the ear regions are configured to generally engage the sides of the wearer's waist and torso, and central region is configured to generally engage the medial portion of the wearer's waist and torso.

Absorbent structure 32 may be manufactured in a wide variety of sizes and shapes (for example, rectangular, trapezoidal, T-shape, I-shape, hourglass shape, etc.) and from a wide variety of materials. The size and the absorbent capacity of absorbent structure 32 should be compatible with the size of the intended wearer and the liquid loading imparted by the intended use of the absorbent article. Further, the size and the absorbent capacity of absorbent structure 32 can be varied to accommodate wearers ranging from infants through adults. In addition, it has been found that with the present invention, the densities and/or basis weights of the respective surge management 46 and retention 48 portions, as well as their relative ratios, can be varied. In a particular aspect of the invention, the absorbent structure has an absorbent capacity of at least about 300 gm of synthetic urine. Alternatively, the absorbent structure can have an absorbent capacity of at least about 400 gm of synthetic urine to provide improved performance.

Various types of wettable, hydrophilic fibrous material can be used to form the component parts of absorbent structure 32. Examples of suitable fibers include naturally occurring organic fibers composed of intrinsically wettable material, such as cellulosic fibers; synthetic fibers composed of cellulose or cellulose derivatives, such as rayon fibers; inorganic fibers composed of an inherently wettable material, such as glass fibers; synthetic fibers made from inherently wettable thermoplastic polymers, such as particular polyester or polyamide fibers; and synthetic fibers composed of a nonwettable thermoplastic polymer, such as polypropylene fibers, which have been hydrophilized by appropriate means. The fibers may be hydrophilized, for example, by treatment with silica, treatment with a material which has a suitable hydrophilic moiety and is not readily removable from the fiber, or by sheathing the nonwettable, hydrophobic fiber with a hydrophilic polymer during or after the formation of the fiber. For the purposes of the present invention, it is contemplated that selected blends of the various types of fibers mentioned above may also be employed.

As used herein, the term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials used for the surge management portion 46 can be provided by a Cahn SFA-222 Surface Force Analyzer System, or a substantially equivalent system. When measured with this system, fibers having contact angles less than 90° are designated "wettable" or hydrophilic, while fibers having contact angles greater than 90° are designated "nonwettable" or hydrophobic.

Retention portion 48 can comprise a matrix of hydrophilic fibers, such as a web of cellulosic fluff, mixed with particles of high-absorbency material. In particular arrangements, retention portion 48 may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness (z-direction) of the absorbent structure, with lower concentrations toward the bodyside of the absorbent structure and relatively higher concentrations toward the outerside of the absorbent structure. Suitable z-gradient configurations are described in U.S. Patent 4,699,823 issued October 13, 1987 to Kellenberger et al.

The superabsorbent particles may also be arranged in a generally discrete layer within the matrix of hydrophilic fibers or may be configured as discrete, separate pocket regions of superabsorbent material. In addition, two or more different types of superabsorbent may be selectively positioned at different locations within or along the fiber matrix.

The high-absorbency material may comprise absorbent gelling materials, such as superabsorbents. The absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces.

Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alpha-olefins, poly(vinyl pyrrolidone), poly(vinylmorpholinone), poly(vinyl alcohol), and mixtures and copolymers thereof. Further polymers suitable for use in the absorbent structure include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be useful in the present invention. Other suitable absorbent gelling materials are disclosed by Assarsson et al. in U.S. Patent 3,901,236 issued August 26, 1975. Processes for preparing synthetic absorbent gelling polymers are disclosed in U.S. Patent 4,076,663 issued February 28, 1978 to Masuda et al. and U.S. Patent 4,286,082 issued August 25, 1981 to Tsubakimoto et al.

Synthetic absorbent gelling materials typically are xerogels which form hydrogels when wetted. The term "hydrogel", however, has commonly been used to also refer to both the wetted and unwetted forms of the material.

As mentioned previously, the high-absorbency material used in retention portion 48 is generally in the form of discrete particles. The particles can be of any desired shape, for example, spiral or semi-spiral, cubic, rod-like, polyhedral, etc. Shapes having a large greatest dimension/smallest dimension ratio, like needles, flakes, and fibers, are also contemplated for use herein. Conglomerates of particles of absorbent gelling material may also be used in retention portion 48.

Preferred for use are particles having an average size of from about 20 microns to about 1 millimeter. "Particle size" as used herein means the weighted average of the smallest dimension of the individual particles.

Suitable high-absorbency materials can have particular characteristics of Absorbent Capacity (sometimes referred to as "AC"), Deformation Under Load (sometimes referred to as "DUL"), and the Wicking Index (sometimes referred to as "WI"). These parameters are described in detail in U.S. Patent Application Serial No. 757,787 of S. Byerly et al., entitled ABSORBENT COMPOSITES AND ABSORBENT ARTICLES CONTAINING SAME and filed on September 11, 1991 (Attorney Docket No. 10,174).

In a particular aspect of the invention, absorbent retention portion 48 comprises a matrix of substantially hydrophilic fibers having a quantity of high-absorbency material distributed therein. Selected superabsorbent polymers having improved absorbent properties can be important for maximizing the performance while retaining the desired thinness of the absorbent article. To provide improved performance, the particles of superabsorbent material can be selected to provide an absorbency-under-load (AUL) value which is within the range of about 25-40, and provide a Absorbent Capacity (AC) value which is within the range of about 32-48. The rate of liquid uptake by the superabsorbent material is within the range of about 3-15 g/g (grams liquid per gram superabsorbent) at 30 seconds of absorbency under load, 6.5-21 g/g at 5 minutes absorbency under load and 25-40 g/g at 60 minutes absorbency under load.

A suitable method for determining AUL is described in detail in U.S. Patent Application Serial No. 184,302 of S. Kellenberger and entitled ABSORBENT PRODUCTS CONTAINING HYDROGELS WITH ABILITY TO SWELL AGAINST PRESSURE (Attorney Docket No. 8786); European Patent Application No. EP 0 339 461 A1, published November 2, 1989.

An example of superabsorbent polymer suitable for use in the present invention is SANWET IM 3900 polymer available from Hoechst Celanese, a business having offices in Portsmouth, Virginia. Other suitable superabsorbents may include W45926 polymer obtained from Stockhausen, a business having offices in Greensboro, North Carolina.

The matrix of hydrophilic fibers comprising retention portion 48 may be a layer of cellulosic wood pulp fluff, and the particles of superabsorbent polymer can be distributed within the matrix of hydrophilic fibers. The hydrophilic fibers and high-absorbency particles can be provided in a fiber-to-particle ratio which is not more than about 75:25, alternatively, is not more than about 70:30, and optionally, is not more than about 55:45, by weight. In further aspects of the invention, the fiber-to-particle ratio is not less than about 25:75, preferably is not less than about 30:70 and more preferably is not less than about 45:55, by weight. Such fiber-to-particle ratios can be particularly desireable in the target zone of the absorbent structure. In particular embodiments of the invention, the fiber-to-particle weight ratio is not more than about 65:35 and is not less than about 50:50 to provide desired performance.

The hydrophilic fibers and high-absorbency particles can form an average composite basis weight which is within the range of about 400-900 gsm. Again, such basis weight is particularly desireable in the target zone of the absorbent structure. In certain aspects of the invention, the average composite basis weight is within the range of about 500-800 gsm, and preferably is within the range of about 550-750 gsm to provide desired performance.

To provide the desired thinness dimension to the various configurations of the absorbent article of the invention, retention portion 48 can be configured with a bulk thickness which is not more than about 0.6 cm. Preferably, the bulk thickness is not more than about 0.53 cm, and more preferably is not more than about 0.5 cm to provide improved benefits. The bulk thickness is determined under a restraining pressure of 1.38 kPa (0.2 psi).

The density of retention portion 48 or other component of the absorbent article can be calculated from its basis weight and thickness. With respect to diapers, for example, the weight and thickness are measured on newly unpacked, unfolded and dry diapers at a restraining pressure of 1.38 kPa (0.2 psi). Conventional thickness measuring devices may be employed to determine the thickness needed to calculate the density.

In the illustrated embodiments of the invention, absorbent retention portion 48 includes 4-22 grams of wood pulp fluff, preferably includes about 8-14 grams of fluff and more preferably includes about 10-12 grams of fluff to provide desired benefits. The wood pulp fluff generally provides shape and form to diaper 10, and carries and positions the particles of superabsorbent polymer or other high-absorbency material. Retention portion 48 can contain about 7-12 grams of superabsorbent polymer, and in the shown embodiment, contains about 8.5 grams of superabsorbent polymer. Sufficient superabsorbent polymer is incorporated into retention portion 48 to provide an adequate total absorbent capacity of at least about 300 gm of urine. For example, a medium size diaper for an infant weighing about 5.9 - 10.4 kg (13 - 23 lb) can typically have a total retention capacity of about 500 grams of urine.

The fluff and superabsorbent particles can be selectively placed into desired zones of retention portion 48. For example, the fluff basis weight may vary across the width dimension of retention portion 48. Alternatively, relatively larger amounts of fluff may be positioned toward the front waistband end of the retention portion. For example, see U.S. Patent 4,585,448 issued April 29, 1986, to K. Enloe. In the illustrated embodiment, the majority of the superabsorbent material may be distributed down a medial region of retention portion 48 which extends along the length dimension of the retention portion and measures about 8.9 - 11.4 cm (3.5 - 4.5 inches) in width. In addition, the superabsorbent material may have a selected zoned placement to reduce the amount of superabsorbent material located proximate the side and end edges of the retention portion. The reduced amounts of superabsorbent material at the edges of the retention portion can improve the containment of the superabsorbent particles within the fibrous fluff matrix of retention portion 48. The pulsed, zoned placement of the superabsorbent material can, for example, be achieved by the method and apparatus described in U.S. Patent 5,028,224 to C. Pieper et al., entitled METHOD AND APPARATUS FOR INTERMITTENTLY DEPOSITING PARTICULATE MATERIAL IN A SUBSTRATE and issued July 2, 1991 (Attorney Docket No. 8761).

In a particular aspect of the invention, absorbent structure 32 can be generally T-shaped with the laterally extending cross-bar of the "T" generally corresponding to the front waistband portion of the absorbent article for improved performance, especially for male infants. In the illustrated embodiments, for example, the retention portion across the ear section of the front waistband region of the article has a cross-directional width of about 22.9 cm (9.0 inches) the narrowest portion of the crotch section has a width of about 8.9 cm (3.5 inches) and the back waistband region has a width of about 11.4 cm (4.5 inches).

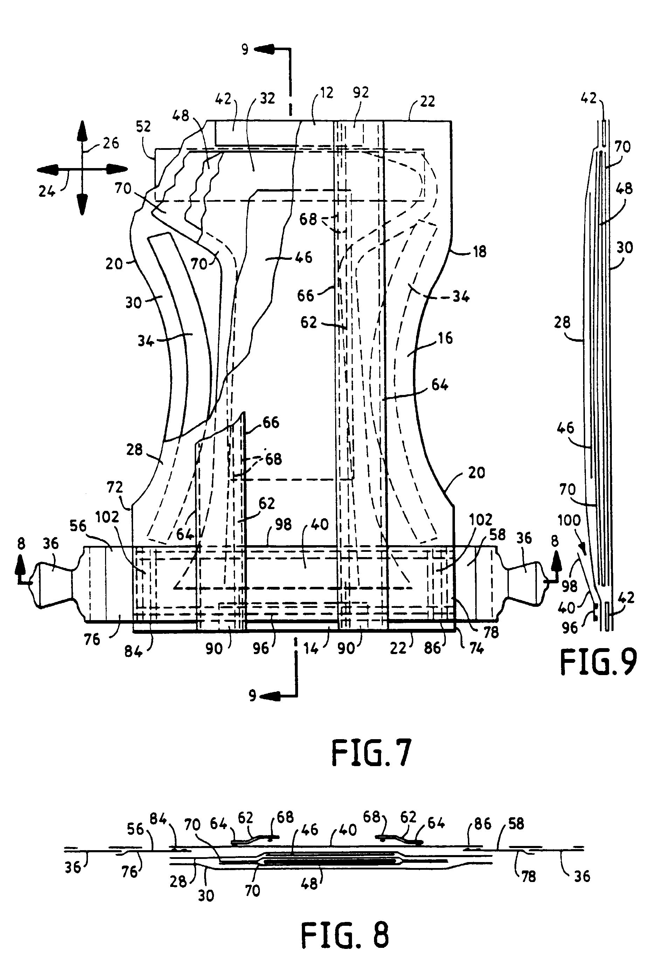

With reference to Fig. 2 and 7, the entire absorbent structure 32, or any individual portion thereof, such as the retention portion, can be overwrapped in a hydrophilic high wet-strength envelope web, such as a high wet-strength tissue or a synthetic fibrous web. Such overwrapping web can also increase the in-use integrity of the absorbent structure. The web can be suitably bonded, such as with adhesive, to absorbent structure 32 and to other components of the product construction.

Due to the high concentrations of superabsorbent particles, or other high-absorbency material, in retention portion 48, there can be an increased difficulty with regard to containing the high-absorbency particles within the retention portion and restricting the movement or migration of the superabsorbent onto the bodyside of the diaper. To improve the containment of the high-absorbency material, absorbent structure 32 can include an improved overwrap, such as wrap sheet 70, placed immediately adjacent and around retention portion 48. The wrap sheet is preferably a layer of absorbent material which covers the major bodyside and outerside surfaces of the retention portion, and preferably encloses substantially all of the peripheral edges of the retention portion to form a substantially complete envelope thereabout. Alternatively, the wrap sheet can provide an absorbent wrap which covers the major bodyside and outerside surfaces of the retention portion, and encloses substantially only the lateral side edges of the retention portion. Accordingly, both the linear and the inwardly curved portions of the lateral side edges of the wrap sheet would be closed about the retention portion. In such an arrangement, however, the end edges of the wrap sheet may not be completely closed around the end edges of the retention portion at the waistband regions of the article.

At least the bodyside layer of wrap sheet 70 has a pore distribution wherein no more than about 5 percent of the pores, as measured by Coulter porometry, are greater than about 50 micrometers in diameter. For example, the complete wrap sheet 70, or at least the bodyside layer of the wrap sheet, may comprise a meltblown web composed of meltblown polypropylene fibers having a fiber size of about 5 micrometers and arranged to form a basis weight within the range of about 8-20 gsm.

Another example of absorbent wrap 70 may comprise a low porosity cellulosic tissue web composed of an approximately 50/50 blend of hardwood/softwood fibers. The tissue has a 5.9 kg (13lb) basis weight at the reel and a porosity of about 969 cfs/m² (90 cfs/sq.ft). Similar to the meltblown wrap sheet material, the entire tissue wrapsheet material, or at least the bodyside layer thereof, has not more than about 5 percent of its pores greater than about 50 micrometers in diameter. Preferably, not more than about 1 percent of the pores are greater than 50 micrometers in diameter.

Absorbent wrap 70 may comprise a multi-element wrapsheet which includes a separate bodyside wrap layer and a separate outerside wrap layer, each of which extends past all or some of the peripheral edges of retention portion 48, as representatively shown in Fig. 2. Such a configuration of the wrap sheet can, for example, facilitate the formation of a substantially complete sealing and closure around the peripheral edges of retention portion 48. In the back waistband portion of the illustrated diaper, the absorbent wrap may also be configured to extend an increased distance away from the periphery of the retention portion to add opacity and strength to the back ear sections of the diaper. In the illustrated embodiment, the bodyside and outerside layers of absorbent wrap 70 extend at least about 1.27 cm (1/2 inch) beyond the peripheral edges of the retention portion to provide an outwardly protruding, flange-type bonding area over which the periphery of the bodyside portion of the absorbent wrap may be completely or partially connected to the periphery of the outerside portion of the absorbent wrap.

The bodyside and outerside layers of wrap sheet 70 may be composed of substantially the same material, or may be composed of different materials. For example, the outerside layer of the wrap sheet may be composed of a relatively lower basis weight material having a relatively high porosity, such as a wet strength cellulosic tissue composed of softwood pulp. The bodyside layer of the wrap sheet may comprise one of the previously described wrap sheet materials which has a relatively low porosity. The low porosity bodyside layer can better prevent the migration of superabsorbent particles onto the wearer's skin, and the high porosity, lower basis weight outerside layer can help reduce costs.

To provide the bonding between the bodyside and outerside portions of absorbent wrap 70, an adhesive, such as National Starch 72-3723 adhesive, can be printed onto the appointed bonding areas 74 of the absorbent wrap with, for example, a rotogravure-type system. The adhesive is available from National Starch and Chemical Co., a business having offices in Bridgewater, New Jersey, and rotogravure-type adhesive applicators are available from Egan Machinery Division, a business having offices at Oconto Falls, Wisconsin. Retention portion 48 can then be placed between the bodyside and outerside portions of absorbent wrap 70, and the mating edges of the absorbent wrap portions can be bonded together to provide a generally complete peripheral seal along substantially the entire perimeter of the retention portion 48. In the illustrated embodiment, the adhesive is applied at an add-on rate of about 5 grams of solids per square meter of bonding to attach together the lapping edges of the bodyside and outerside portions of absorbent wrap 70.

With alternative arrangements having an absorbent wrap composed of a nonwoven meltblown fibrous web, the peripheral sealing of the bodyside and outerside wrap layers may be accomplished by employing hot calendering to provide a sealed strip region around the periphery of the retention portion.

Due to the thinness of retention portion 48 and the high superabsorbent concentrations within the retention portion, the liquid uptake rates of the retention portion, by itself, may be too low, or may not be adequately sustained over three insults of liquid into the absorbent structure. The addition of a porous, liquid-permeable layer of surge management material, however, can advantageously improve the overall uptake rate of the composite absorbent structure. Surge management portion 46 is typically less hydrophilic than retention portion 48, and has an operable level of density and basis weight to quickly collect and temporarily hold liquid surges, to transport the liquid from its initial entrance point and to substantially completely release the liquid to other parts of the absorbent structure 32, particularly retention portion 48. This configuration can help prevent the liquid from pooling and collecting on the portion of the absorbent garment positioned against the wearer's skin, thereby reducing the feeling of wetness by the wearer.

Various woven and nonwoven fabrics can be used to construct surge management portion 46. For example, the surge management portion may be a layer composed of a meltblown or spunbonded web of polyolefin fibers.

The surge management layer may also be a bonded-carded-web or an airlaid web composed of natural and synthetic fibers. The bonded-carded-web may, for example, be a powder-bonded-carded web, an infrared bonded carded web, or a through-air-bonded-carded web. The infrared and through-air bonded carded webs can optionally include a mixture of different fibers, and the fiber lengths within a selected fabric web may be within the range of about 2.54-7.62 cm (1.0-3.0 inch). The surge management portion may be composed of a substantially hydrophobic material, and the hydrophobic material may optionally be treated with a surfactant or otherwise processed to impart a desired level of wettability and hydrophilicity.

Surge management portion 46 can have a generally uniform thickness and cross-sectional area. Alternatively, a configuration can be used wherein the bodyside surface area of the surge management portion is greater or less than the surface area of a section taken along an X-Y plane located below the bodyside surface of the surge management portion.

The representative diaper 10 can include a surge management portion 46 which is arranged in a direct, contacting liquid communication with an adjacent absorbent retention portion 48. As representatively shown, surge management portion 46 may be configured for placement adjacent an outwardly facing, outerside of topsheet 28. Optionally, the surge management portion can be placed adjacent an inwardly facing, bodyside surface of topsheet layer 28. The shown configuration of the surge management portion is operably connected to the topsheet layer with a conventional pattern of adhesive, such as a swirl adhesive pattern. In addition, the surge management portion can be operably connected to the bodyside layer of wrapsheet 70 with a conventional pattern of adhesive. The amount of adhesive add-on should be sufficient to provide the desired levels of bonding, but should be low enough to avoid excessively restricting the movement of liquid from the topsheet layer, through the surge management portion and through the wrapsheet layer.

In the various embodiments of the invention, at least a major part of surge management portion 46 is located within target zone 44, and optionally, the surge management portion can have an areal extent which extends completely over target zone 44. Retention portion 48 is positioned in liquid communication with surge management portion 46 to receive liquids released from the surge management portion, and to hold and store the liquid. In the shown embodiments, surge management portion 46 comprises a separate layer which is positioned over another, separate layer comprising the retention portion, thereby forming a dual-layer arrangement. The surge management portion serves to quickly collect and temporarily hold discharged liquids, to transport such liquids from the point of initial contact and spread the liquid to other parts of the surge management portion, and then to substantially completely release such liquids into the layer or layers comprising retention portion 48.

The representatively shown configuration of the surge management portion is substantially free of absorbent gelling material. Surge management portion 46 may, however, contain a very small amount of particulate gelling material to help acquire an initial liquid surge, but the amount should not be excessive. When excessive amounts of particulate absorbent gelling material are maintained in target zone 44, however, the particles can cause the structure to retain and hold unacceptably high amounts of the liquid. In addition, the transport of liquids away from target zone 44 to other sections of absorbent structure 32, particularly retention portion 48, can be undesirably impaired.

As mentioned previously, surge layer 46 can be a separately formed layer, which lies adjacent the outwardly facing surface of topsheet 28 between the retention portion and topsheet. Thus, surge management portion 46 need not comprise the entire thickness of absorbent structure 32. The retention portion can optionally include a recess area which wholly or partially surrounds surge management portion 46, or the retention portion can be entirely positioned below the surge management portion. The arrangement which includes the recess in retention portion 48 can advantageously increase the area of contact and liquid communication between the retention portion and surge management portion 48. It should be understood, however, that surge management portion 46 could optionally be constructed to extend through the entire thickness of absorbent structure 32 so that the capillary flow of liquid into retention portion 48 occurs primarily in a generally sideways (X-Y) direction.

A capillary force differential created at the interface between the retention portion 48 and the material immediately adjacent the bodyside of the retention portion can improve the containment characteristics of absorbent structure 32. For example, if the surge management portion is composed of layer 46 positioned immediately adjacent to the retention portion, and if the surge layer is appropriately configured to provide and maintain a relatively lower capillary attraction, as compared to the capillary attraction exhibited by retention portion 48, then liquid surges occurring in target zone 44 tend to be desorbed more readily from the surge management portion and into the retention portion. Because retention portion 48 can thereby have a relatively higher capillarity than surge management portion 46, the liquid surges tend to be drawn into retention portion 48 and distributed to the more remote regions thereof by wicking along the plane generally defined by the retention portion.

The surge management portion can be of any desired shape consistent with the absorbency requirements of absorbent structure 32. Suitable shapes include for example, circular, rectangular, triangular, trapezoidal, oblong, dog-boned, hourglass-shaped, or oval. Preferred shapes of the surge management portion are those that increase the contacting, liquid communicating surface area between surge management portion 46 and retention portion 48 so that the relative capillarity difference between the portions can be fully utilized. In certain embodiments, for example, the surge management portion can be generally rectangular-shaped.

In the various configurations of the invention, surge management portion 46 may extend over the complete length of retention portion 48, or may extend over only a part of the retention portion length. Where the surge management portion extends only partially along the length of the retention portion, the surge management portion may be selectively positioned anywhere along absorbent structure 32. For example, surge management portion 46 may function more efficiently when it is offset toward the front waistband of the garment and transversely centered within a front section of absorbent structure 32. Thus, surge management portion 46 can be approximately centered about the longitudinal center line of absorbent structure 32, and positioned primarily in a central region of a front section of the absorbent structure 32.

In other aspects of the invention, the end edges of the surge management portion can be spaced longitudinally inboard from the end edges of the retention portion 48. In particular configurations of the invention, the corresponding, relatively adjacent front end edge of surge management portion 46 can be spaced a predetermined discrete distance from a front waistband end edge of the retention portion 48.

It has been found that an effective fabric for constructing the surge management portion can be distinctively characterized by particular parameters. Such parameters include, for example, basis weight, permeability, porosity, surface area per void volume (SA/VV), compression resiliency and saturation capacity. Further parameters can include a bonding matrix which will help stabilize the pore size structure, and hydrophilicity. The bond-matrix and the blend of fiber deniers can advantageously provide for and substantially maintain a desired pore size structure.

Additional details regarding the surge materials and suitable techniques for determining the above-described parameters are set forth in U.S. Patent Application Serial No. 206,986 of C. Ellis and D. Bishop, entitled, FIBROUS NONWOVEN WEB SURGE LAYER FOR PERSONAL CARE ABSORBENT ARTICLES AND THE LIKE, and filed March 4, 1994 (Attorney docket No. 11,256); and U.S. Patent Application Serial No. 206,069 of C. Ellis and R. Everett, entitled, IMPROVED SURGE MANAGEMENT FIBROUS NONWOVEN WEB FOR PERSONAL CARE ABSORBENT ARTICLES AND THE LIKE, and filed March 4, 1994 (Attorney docket No. 11,387).

In particular configurations of the invention, the surge material can include bicomponent fibers. For example, polypropylene/polyethylene bicomponent fibers may be employed to form the bicomponent fiber portion of any of the described fabrics. In addition, the bicomponent fibers may be flat crimped or helically crimped.

In particular aspects of the invention, the fibrous nonwoven web comprising surge management portion 46 can be a bonded, uniformly mixed, single layer structure having a basis weight of at least 20 grams per square meter, a void volume between about 40 and 60 cubic centimeters per gram of web at a pressure of 68.9 Pa (689 dynes per square meter), a permeability of about 5,000 to about 8,000 darcy, a porosity of about 97.2% to about 98.8% and a surface area per void volume of about 24 to about 49 square centimeters per cubic centimeters. The web fibers may be thermoplastic, and may be heat bonded to one another. In addition, the web structure can have a density within a range of about 0.017-0.025 gm/cc, as determined at a pressure of 68.9 Pa (689 dynes per square meter).

In other aspects of the invention, the fibrous nonwoven web can be made from or include a plurality of fibers bonded to one another to form a lofty nonwoven web having a basis weight of at least 20 grams per square meter. In more refined embodiments, the basis weight can range from about 40 to about 68 grams per square meter. The web can be made entirely from bicomponent fibers which are typically crimped and which will generally have a fiber denier equal to or greater than 2 denier. Alternatively, the web can be made from a combination of fibers such as bicomponent fibers and polyester fibers. In such embodiments, the web will usually include at least 50 percent by weight of bicomponent fibers. The resultant web will have a void volume of between about 80 and about 117 cubic centimeters per gram of web at 68.9 Pa (689 dynes per square meter) pressure, a permeability of about 8,000 to about 15,000 darcy, a porosity of about 98.6 to about 99.4 percent, a surface area per void volume of about 10 to about 25 square centimeters per cubic centimeter, a saturation capacity between about 55 and about 80 grams of 0.9 percent saline solution per gram of web and a compression resilience in both the wet and dry state of at least 60 percent. In addition, the web structure can have a density within a range of about 0.008-0.013 gm/cc, as determined at a pressure of 68.9 Pa (689 dynes per square meter).

The basis weight of surge portion 46 can be important for providing a total holding capacity which is adequate to temporarily retain the amount of liquid that is typically discharged by a wearer during a single surge/insult of liquid into the absorbent article. It will be readily apparent that absorbent articles requiring more surge capacity may also require proportionally greater amounts of surge management material. The surge management material, however, need not be of uniform basis weight throughout its areal extent, but instead can be arranged so that some sections have more surge management material compared to other sections. For the purposes of the present invention, the effective basis weight will be the weight of the surge management material divided by the area over which the surge management portion extends. The surge management material employed with the present invention will be at least about 20 grams per square meter with no real upper limit, with the target range being from about 40 to about 68 grams per square meter.

To ensure rapid intake of liquid, the overall structure of the surge portion 46 should have hydrophilic tendencies. At least a portion of the fibers should have a contact angle less than 90 degrees. As a result, the fibrous nonwoven web will have sufficient hydrophilic tendencies when the web has a saturation capacity greater than 55 grams of 0.9% saline solution per gram of web.

Another important feature of the surge material employed with the present invention is its resiliency in both the wet and dry states. A unique feature of the surge material is the amount of liquid which the material is able to absorb upon rapid insult. In addition, once the liquid has been absorbed, the surge material does not readily collapse. Excessive collapse would be detrimental to the overall performance of the material in that the collapsing of the material would result in a reduced capacity for retaining liquid. Surge materials employed with the present invention should have compression resilience values in both the wet and dry states of at least about 60%.

The distinctive permeability, specific volume, porosity, and ratio of surface area to void volume parameters within the surge management portion of the invention can advantageously provide for a sufficiently rapid uptake of the liquid surges delivered onto the target zone, and also allow a controlled spreading of the liquid through the void volume of its structure to temporarily fill it. Over a relatively short period of time, the surge management portion can then be desorbed through the cooperative operation of the underlying or otherwise adjacent liquid retention portion.

The surge management portion is configured to cooperate with the other diaper components, such as top sheet 28 and retention portion 48, to provide for a rapid uptake of liquid discharges from the wearer. It is appreciated that a surge material with relatively small pore sizes may exhibit a rate of liquid penetration into the retention portion which may be too slow. A layer of surge management material having relatively large pore sizes, however, may provide insufficient restriction to sideways movement of liquid through the material along the plane of the material layer. As a result, the liquid may run off to the sides of the layer and leak from the article before the absorbent retention material can gather and contain the liquid. Such undesired, excessive run off may become more apparent when the absorbent material has already absorbed one or more previous discharges of liquid.

To help reduce the occurrence of excessive run off, the surge management portion present invention can be configured to have edge barriers constructed along selected edge regions thereof. In particular aspects, the edge barriers can be provided for by separate layers of barrier material or by edge regions which are otherwise configured to include relatively small pores therein. The small pore regions can be located along the end edge regions and/or side edge regions of the surge management portion, and are configured to be sufficiently continuous to provide operable barriers to the sideways movement of liquid.

In the various configurations of the invention, the edge barrier may be provided at only the end edges 88 of the surge material. Alternatively, the edge barrier may be provided at only the side edges 84 and 86 of the surge material, and optionally may be provided at both the end and side edges of the surge material to provide desire performance.

In the shown configuration, side panel members 56 and 58 are separate members operably connected and attached to laterally opposed end sections of the back waistband portion of backsheet 30. The side panels are composed of a substantially elastomeric material, such as a stretch-bonded-laminate (SBL) material, a neck-bonded-laminate (NBL) material, an elastomeric film, an elastomeric foam material, or the like. For example, suitable meltblown elastomeric fibrous webs are described in U.S. Patent 4,663,220 issued May 5, 1987 to T. Wisneski et al. Examples of composite fabrics comprising at least one layer of nonwoven textile fabric secured to a fibrous elastic layer are described in European Patent Application No. EP 0 110 010 published on April 8, 1987 with the inventors listed as J. Taylor et al. Examples of NBL materials are described in U.S. Patent 5,226,992 issued July 13, 1993 to Mormon.

The fastening means, such as provided by tape tab fasteners 36, are typically applied to the side panels 56 and 58 of diaper 10 to provide a mechanism for holding the diaper on the wearer. The tab fasteners 36 can be any of those well known in the art, and are typically applied to the corners of diaper 10. For example, adhesive fasteners, mechanical fasteners, hook and loop fasteners, snaps, pins or buckles, may be used alone, or in combination. In the shown configuration, the fasteners are adhesive fasteners, which are constructed to releasably adhere to a landing zone patch attached to the front waistband section of the diaper to provide a refastenable adhesive fastening system. More particularly, the fastener tabs 36 connect to associated, laterally outboard edge regions of the side panels 56 and 58 along an appointed factory-bond region of the tab fasteners. In particular aspects of the invention, the fastener tabs can have a relatively wide user-bond section in combination with a relatively narrower intermediate section. The intermediate section is positioned between the user-bond and factory-bond sections of the fastener tab.

Articles which include elastomeric side panels and distinctively configured fasteners are described in U.S. Patent Application Serial No. 168,615 of T. Roessler et al., entitled DYNAMIC FITTING DIAPER and filed December 16, 1993 (Attorney docket No. 10,961).

The fastening systems can include a stress beam member for distributing applied stresses the area of the side panel material, and can include fastening tabs which incorporate a necked down intermediate region in combination with a relatively wider, user-bond section thereof. Techniques for forming the desired fastening systems are described in U.S. Patent Application Serial No. 200,593 of T. Roessler et al., entitled METHOD FOR MAKING A FASTENING SYSTEM FOR A DYNAMIC FITTING DIAPER and filed February 23, 1994 (Attorney docket No. 11,186).

With reference to Fig. 1, bridge member 40 is a component which is separate from backsheet layer 30 and topsheet layer 28. In addition, the bridge member is a component which is separate from each of the side panels 56 and 58. The bridge member 40 extends laterally along the cross-direction 24 of the article, and operably interconnects between the pair of side panels 56 and 58. More particularly, bridge member 40 includes opposed lateral end sections 84 and 86, with elasticized side panel 56 operably attached to end section 84 and with elasticized side panel 58 operably attached to lateral end section 86. The bridge member provides a means for defining and maintaining a desired lateral separation and relative lateral positioning and alignment between the elasticized side panels 56 and 58. Accordingly, the bridge member and side panels form an integrated sub-assembly which can be more reliably positioned at a desired location and orientation within the final article, and can more effectively avoid undesired offsets and non-alignments. The bridge member 40 can more effectively maintain desired characteristics of cooperation between the bridge member and side panels, and can reliably hold the side panels members in the desired spacing and alignment during high speed manufacturing processes. In addition, the composition of the bridge member material and the relative positioning of the bridge member component, with respect to the backsheet and topsheet layers, can be selectively configured to provide distinctive advantages.

As representatively shown in Fig. 1, bridge member 40 has a longitudinal length 80 which is less than the longitudinal length of backsheet 30. In addition, the bridge member longitudinal length 80 can be less than the longitudinal length of topsheet 28. In particular aspects of the invention, bridge member length 80 is not less than about 2 centimeters. The bridge member length is alternatively not less than about 3 centimeters, and is optionally not less than about 5 centimeters. In other aspects of the invention, the bridge member length 80 is not more than about 25 centimeters. Alternatively, the bridge member length is not more than about 18 centimeters, and optionally is not more than about 10 centimeters to provide desired performance.

In the various aspects of the invention, the bridge member can also be selectively positioned relative to the backsheet and topsheet layers to provide differing performance advantages. With reference to Figs. 1-3, for example, the bridge member can be located in a laminated configuration onto an outwardly facing, major surface of backsheet layer 30. As a result, the bridge member can provide a more rigid, belt-type function and can help provide improved waist fit and reduced leakage.

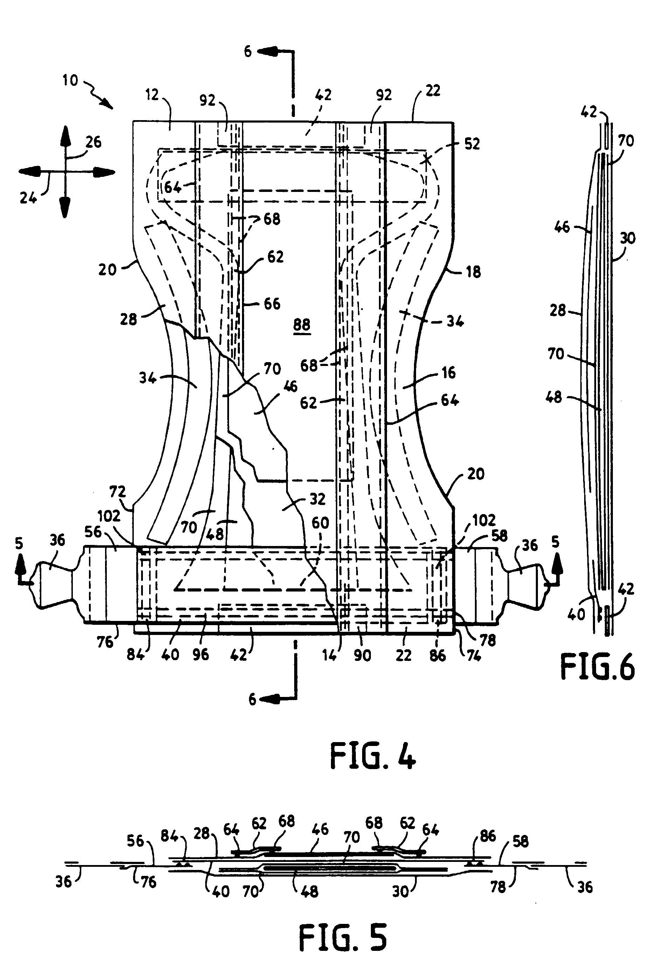

With reference to an alternative configuration representatively shown in Figs. 4-6, bridge member 40 can be sandwiched between backsheet 30 and topsheet 28, and can be arranged with at least a portion of the bridge member located in a generally adjacent, overlapping position to cover an inward bodyside surface 54 of absorbent body 32. As a result, the bridge member can provide more consistent waist fit and an internal "dam" to help reduce leakage. The bridge member can have a laterally extending fixed edge region 96, and a pair of laterally opposed, longitudinally extending, fixed side regions 102 to provide a more effective dam structure. In addition, the fixed edge regions 96 and 102 can be attached to backsheet 30 and topsheet 28 and configured to provide an operable seal which can substantially prevent undesired leakage of liquid past the fixed edge attachments.

The bridge member 40 can alternatively be positioned to overlie onto a bodyside surface 88 of topsheet 28 and cooperate with the generally longitudinally extending containment flaps 62 to provide further advantages. With reference to Fig. 7-9, the bridge member can be interposed between the topsheet and containment flaps. The longitudinally spaced end sections 90 and 92 of containment flaps 62 are oriented generally parallel to a plane generally defined by topsheet 28, and may be operably attached to the bridge member or topsheet to maintain the laid-down configuration. In the shown embodiment, for example, bridge member 40 includes a longitudinally outboard fixed edge region 96, laterally outboard fixed edge regions 102, and a movable edge region 98. Fixed edge region 96 is operably attached to the immediately contacting portions of topsheet 28 and containment flaps 62. In particular configurations, the attachments of fixed edge regions 96 and 102 are constructed to provide an operable seal which can substantially prevent undesired leakage of liquid past the fixed edge attachments.

At least a portion of the Inboard edge region 98 of bridge member 40 is movable. In the shown embodiment, for example, a medial section of inboard edge 98 is movable while the lateral end sections of the inboard edge are attached to the corresponding, immediately adjacent sections of topsheet 28. As a result, the movable portion of the inboard edge 98 can cooperatively provide a containment pocket 100 at the waistband section of the article.

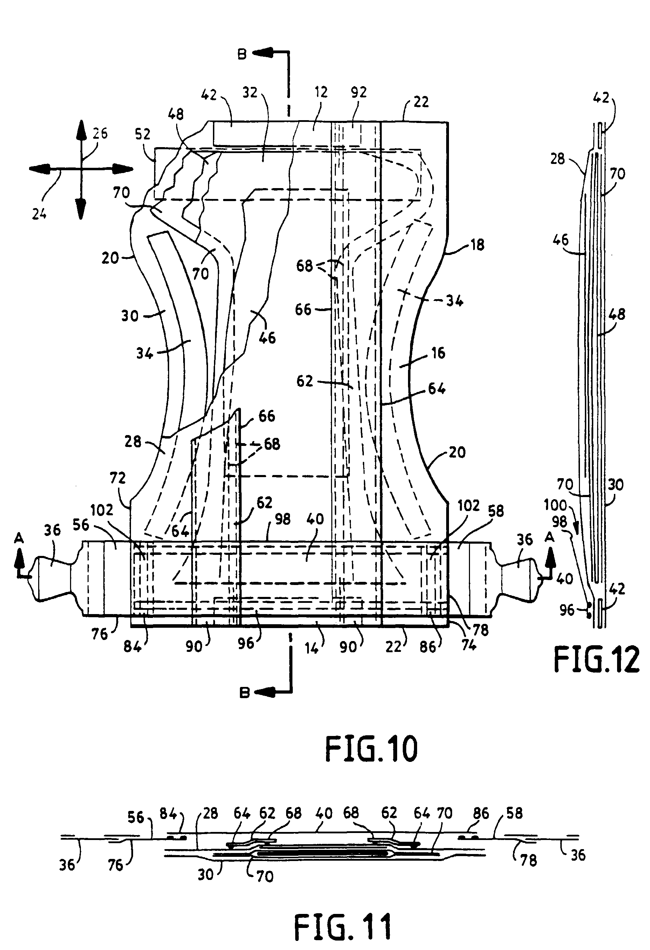

The bridge member 40 can optionally be positioned to overlie onto a bodyside surface 88 of topsheet 28 and further cooperate with the generally longitudinally extending containment flaps 62 to provide additional advantages. With reference to Fig. 10-12, the longitudinally spaced end sections 90 and 92 of containment flaps 62 are oriented generally parallel to a plane generally defined by topsheet 28, and may be operably attached to the topsheet to maintain the laid-down configuration. Bridge member 40 Is arranged to span over the containment flaps 62. Accordingly, a bodyside surface 94 of bridge member 40 is appointed for placement against the wearer. With reference to Figs. 10 and 11, bridge member 40 includes a longitudinally outboard fixed edge region 96, laterally outboard fixed edge regions 102, and a movable edge region 98. Fixed edge region 96 is operably attached to the immediately contacting portions of topsheet 28 and containment flaps 62. In particular configurations, the attachments of fixed edge regions 96 and 102 are constructed to provide an operable seal which can substantially prevent undesired leakage of liquid past the fixed edge attachments.

At least a portion of the inboard edge region 98 of bridge member 40 is movable. In the shown embodiment, for example, a medial section of inboard edge 98 is movable while the lateral end sections of the inboard edge are attached to the corresponding, immediately adjacent sections of topsheet 28 and containment flaps 62. In particular configurations, the movable portion of inboard edge 98 spans between the generally movable, distal edges 66 of the containment flaps 62. As a result, the movable portion of the inboard edge 98 can cooperatively provide a containment pocket 100 at the waistband section of the article.

In particular aspects of the invention, bridge member 40 can be constructed of a substantially nonwettable, hydrophobic material. The bridge member material can also be substantially impermeable to a passage of liquid through its thickness. In other configurations, the bridge member can be constructed of a material which is permeable to a passage of gas, such as air, therethrough.

Accordingly, where the bridge member is capable of restricting the passage of liquid therethrough, the configuration where a portion of bridge member 40 overlaps onto the bodyside surface 54 of the absorbent body can provide a dam-type structure which can operably restrict a leakage of liquid past the waistband portion of the article. The portion of bridge member 40 which is connected and laminated between backsheet 30 and topsheet 28 is suitably bonded and sealed to operably restrict the leakage of liquid through the laminated structure.

In other aspects of the invention, the bridge member material may be elastomeric or substantially nonelastomeric. The representatively shown configuration, for example, can include a bridge member material composed of a fibrous material which is similar to the material comprising topsheet 28. Other conventional materials, such as polymer films, may also be employed. In other aspects of the invention, bridge member 40 is constructed of a material which is permeable to gas, such as ambient air. Alternative configurations of the invention can include a bridge member which is constructed of a material which is resistant to a passage of aqueous liquid, such as urine, therethrough. For example, bridge member 40 may be constructed of a spunbond-meltblown-spunbond (SMS) laminate material. In the illustrated embodiment, for example, the bridge member can be constructed of a SMS material having a basis weight of about 0.85 gsm (about 28 osy). The spunbond layers are composed of polypropylene fibers, and the meltblown layer is composed of meltblown polypropylene fibers.

In the various configurations of the invention, such as where the bridge member 40 is configured to be permeable to gas while having a resistance and limited permeability to aqueous liquid, the liquid resistent material can have a construction which is capable of supporting a hydrohead of at least about 45 cm of water substantially without leakage therethrough. A suitable technique for determining the resistance of a material to liquid penetration is Federal Test Method Standard FTMS 191 Method 5514, dated 31 December 1968.

## Claims

1. An absorbent article (10) having a front waistband section (12), a rear waistband section (14) and an intermediate section (16) which interconnects said front and rear waistband sections, said article comprising:
a backsheet layer (30) having a laterally extending width and a longitudinally extending length;
a liquid permeable topsheet layer (28) superposed on said backsheet layer (30), said topsheet layer (28) having a laterally extending width and a longitudinally extending length;
an absorbent body (32) located between said backsheet layer (30) and said topsheet layer (28);
a bridge member (40) which is provided separate from said backsheet (30) and topsheet (28) layers, at least a portion of said bridge member (40) arranged in an overlapping relation with a longitudinal end section of said absorbent body (32) and connected to at least one of said backsheet (30) and topsheet (28) layers; elasticized side panels (56, 58) constructed to be elastically stretchable at least along a lateral cross-direction of said article (10);
and
fastening means (36) for securing said article (10) on a wearer, said fastening means (36) being connected to a laterally distal end region of at least one of said side panels (56, 58);
said article characterised in that said elasticized side panels (56, 58) are provided separate from said bridge member and are connected to said bridge member (40) at each laterally opposed end section (84, 86) of said bridge member (40);
said bridge member (40) extends laterally to interconnect between said elasticized side panels (56, 58);
said bridge member (40) is configured to form an integrated subassembly with said side panels, said subassembly maintaining a desired lateral separation and alignment between said separately provided side panels (56, 58) when said sub-assembly is positioned in said article during manufacturing.

2. An absorbent article (10) as recited in claim 1, wherein said bridge member (40) has a longitudinal length which is less than said backsheet length and less than said topsheet length.

3. An absorbent article (10) as recited in claim 2, wherein said bridge member (40) is connected between said backsheet (30) and topsheet (28) layers and is arranged to overlap adjacent to an inward, bodyside surface of said absorbent body (32).

4. An absorbent article (10) as recited in claim 3, wherein said bridge member (40) has a laterally extending fixed edge region (96), and a pair of laterally opposed, longitudinally extending, fixed side regions (102).

5. An absorbent article (10) as recited in claim 4, wherein said bridge member (40) is constructed to substantially prevent a passage of liquid through a thickness thereof.

6. An absorbent article (10) as recited in claim 5, wherein said bridge member (40) is permeable to a passage of gas therethrough.

7. An absorbent article (10) as recited in claim 2, wherein said bridge member (40) is connected in an adjacent facing relation with a major, bodyside surface of said topsheet layer (28).

8. An absorbent article (10) as recited in claim 7, wherein said bridge member (40) is constructed to substantially prevent a passage of liquid through its thickness.

9. An absorbent article (10) as recited in claim 8, further comprising a pair of longitudinally extending containment flaps (62), wherein said bridge member (40) is interposed between said containment flaps (62) and said topsheet layer (28).

10. An absorbent article (10) as recited in claim 9, wherein said bridge member (40) has a laterally extending fixed edge region (96), and a pair of laterally opposed, longitudinally extending, fixed side regions (102).

11. An absorbent article (10) as recited in claim 10, wherein said laterally extending fixed edge region (96) and said longitudinally extending, fixed side regions (102) of said bridge member are attached to provide operable seals which can substantially prevent undesired leakage of liquid past said fixed edge regions.

12. An absorbent article (10) as recited in claim 2, wherein said article further comprises a pair of longitudinally extending containment flaps (62) and said bridge member (40) is connected to span over said containment flaps (62) and to provide a bodyside surface for contacting the wearer.

13. An absorbent article (10) as recited in claim 12, wherein said bridge member (40) has a laterally extending fixed edge region, and a pair of laterally opposed, longitudinally extending, fixed side regions.

14. An absorbent article (10) as recited in claim 13, wherein said bridge member (40) is constructed to substantially prevent a passage of liquid through its thickness.

15. An absorbent article (10) as recited in claim 14, wherein said bridge member (40) is gas permeable.

16. An absorbent article (10) as recited in claim 15, wherein said fastening means (36) includes a first fastener connected to a laterally distal end region of a first side panel (56) and a second fastener connected to a laterally distal end region of a second side panel (58).

17. An absorbent article (10) as recited in claim 15, wherein said first and second fasteners include adhesive user-bond regions.

18. An absorbent article (10) as recited in claim 15, wherein said first and second fasteners include mechanical user-bond regions.

## Patentansprüche

1. Saugfähiger Artikel (10) mit einem vorderen Taillenbundabschnitt (12), einem hinteren Taillenbundabschnitt (14) und einem dazwischenliegenden Abschnitt (16), der den vorderen und den hinteren Taillenbundabschnitt miteinander verbindet, wobei der Artikel umfasst:
eine Rückschichtlage (30) mit einer sich lateral erstreckenden Breite und einer sich in Längsrichtung erstreckenden Länge;
eine flüssigkeitsdurchlässige Deckschichtlage (28), die auf der Rückschichtlage (30) liegt, wobei die Deckschichtlage (28) eine sich lateral erstreckende Breite und eine sich in Längsrichtung erstreckende Länge aufweist;
einen Saugkörper (32), der zwischen der Rückschichtlage (30) und der Deckschichtlage (28) angeordnet ist;
ein Brückenelement (40), das getrennt von der Rückschichtlage (30) und der Deckschichtlage (28) bereitgestellt ist, wobei zumindest ein Teil des Brückenelements (40) mit einem längsseitigen Endabschnitt des Saugkörpers (32) in einer überlappenden Beziehung angeordnet ist und zumindest an einer der Lagen der Rückschicht (30) oder Deckschicht (28) befestigt ist; elastifizierte Seitenteile (56, 58), die so gestaltet sind, dass sie zumindest entlang einer seitlichen Querrichtung des Artikels (10) elastisch dehnbar sind;
und Befestigungsmittel (36), um den Artikel (10) an einem Träger zu sichern, wobei die Befestigungsmittel (36) an einem lateral distalen Endbereich von zumindest einem der Seitenteile (56, 58) befestigt sind;
wobei der Artikel dadurch gekennzeichet ist, dass die elastifizierten Seitenteile (56, 58) getrennt von dem Brückenelement (40) bereitgestellt sind und mit dem Brückenelement (40) an jedem der lateral gegenüberliegenden Endabschnitte (84, 86) dieses Brückenelements (40) befestigt sind;
wobei das Brückenelement (40) sich so in Längsrichtung erstreckt, dass es die elastifizierten Seitenteile (56, 58) in dazwischenliegender Weise miteinander verbindet;
wobei das Brückenelement (40) so gestaltet ist, dass es mit den Seitenteilen eine integrierte Unteranordnung bildet, wobei die Unteranordnung eine gewünschte laterale Abtrennung und Ausrichtung zwischen den getrennt bereitgestellten Seitenteilen (56, 58) aufrechterhält, wenn die Unteranordnung während der Herstellung in dem Artikel angeordnet wird.

2. Saugfähiger Artikel (10) gemäß Anspruch 1, wobei das Brückenelement (40) eine in Längsrichtung verlaufende Länge aufweist, die geringer ist als die Länge der Rückschicht und die Länge der Deckschicht.

3. Saugfähiger Artikel (10) gemäß Anspruch 2, wobei das Brückenelement (40) zwischen der Rückschicht (30) und der Deckschicht (28) verbunden und so angeordnet ist, dass es benachbart zu einer inneren, körperseitigen Oberfläche des saugfähigen Körpers (32) überlappt.

4. Saugfähiger Artikel (10) gemäß Anspruch 3, wobei das Brückenelement (40) einen sich lateral erstreckenden, feststehenden Randbereich (96) aufweist sowie ein Paar lateral gegenüberliegende, sich in Längsrichtung erstreckende, feststehende Seitenbereiche (102).

5. Saugfähiger Artikel (10) gemäß Anspruch 4, wobei das Brückenelement (40) so gestaltet ist, dass es ein Durchsickern von Flüssigkeit durch eine Dicke davon im Wesentlichen verhindert.

6. Saugfähiger Artikel (10) gemäß Anspruch 5, wobei das Brückenelement (40) für das Durchströmen von Gas durchlässig ist.

7. Saugfähiger Artikel (10) gemäß Anspruch 2, wobei das Brückenelement (40) so mit einer körperseitigen Hauptoberfläche der Deckschichtlage (28) verbunden ist, dass es ihr zugewandt ist und ihr benachbart ist.

8. Saugfähiger Artikel (10) gemäß Anspruch 7, wobei das Brückenelement (40) so gestaltet ist, dass es ein Durchsickern von Flüssigkeit durch seine Dicke im Wesentlichen verhindert.

9. Saugfähiger Artikel (10) gemäß Anspruch 8, weiter umfassend ein Paar sich in Längsrichtung erstreckende Einschlussklappen (62), wobei das Brückenelement (40) zwischen den Einschlussklappen (62) und der Deckschichtlage (28) angeordnet ist.

10. Saugfähiger Artikel (10) gemäß Anspruch 9, wobei das Brückenelement (40) einen sich lateral erstreckenden, feststehenden Randbereich (96) aufweist sowie ein Paar lateral gegenüberliegende, sich in Längsrichtung erstreckende, feststehende Seitenbereiche (102).

11. Saugfähiger Artikel (10) gemäß Anspruch 10, wobei der sich lateral erstreckende, feststehende Randbereich (96) und die sich in Längsrichtung erstreckenden, feststehenden Seitenbereiche (102) des Brückenelements so befestigt sind, dass sie operable Dichtungen bereitstellen, die unerwünschtes Auslaufen von Flüssigkeit über die feststehenden Randbereiche im Wesentlichen verhindern können.

12. Saugfähiger Artikel (10) gemäß Anspruch 2, wobei der Artikel weiter ein Paar sich in Längsrichtung erstreckende Einschlussklappen (62) umfasst und das Brückenelement (40) so befestigt ist, dass es die Einschlussklappen (62) überspannt und dass es eine körperseitige Oberfläche bereitstellt, um den Träger zu berühren.

13. Saugfähiger Artikel (10) gemäß Anspruch 12, wobei das Brückenelement (40) einen sich lateral erstreckenden, feststehenden Randbereich aufweist sowie ein Paar lateral gegenüberliegende, sich in Längsrichtung erstreckende, feststehende Seitenbereiche.

14. Saugfähiger Artikel (10) gemäß Anspruch 13, wobei das Brückenelement (40) so gestaltet ist, dass es ein Durchsickern von Flüssigkeit durch seine Dicke im Wesentlichen verhindert.

15. Saugfähiger Artikel (10) gemäß Anspruch 14, wobei das Brückenelement (40) gasdurchlässig ist.

16. Saugfähiger Artikel (10) gemäß Anspruch 15, wobei die Befestigungsmittel (36) ein erstes Befestigungselement, das mit einem lateral distalen Endbereich eines ersten Seitenteils (56) verbunden ist, und ein zweites Befestigungselement, das mit einem lateral distalen Endbereich eines zweiten Seitensteils (58) verbunden ist, umfassen.

17. Saugfähiger Artikel (10) gemäß Anspruch 15, wobei das erste und zweite Befestigungselement Benutzerbindungsklebebereiche umfassen.

18. Saugfähiger Artikel (10) gemäß Anspruch 15, wobei das erste und zweite Befestigungselement mechanische Benutzerbindungsbereiche umfassen.

## Revendications

1. Article absorbant (10) ayant une section de ceinture avant (12), une section de ceinture arrière (14) et une section intermédiaire (16) qui interconnecte lesdites sections de ceinture avant et arrière, ledit article comprenant :
une couche de feuille support (30) ayant une largeur s'étendant transversalement et une longueur s'étendant longitudinalement ;
une couche de feuille supérieure (28) perméable aux liquides, superposée à ladite couche de feuille support (30), ladite couche de feuille supérieure (28) ayant une largeur s'étendant transversalement et une longueur s'étendant longitudinalement ;
un corps absorbant (32) situé entre ladite couche de feuille support (30) et ladite couche de feuille supérieure (28) ;
un élément de pontage (40) fourni séparé de ladite couche de feuille support (30) et de ladite couche de feuille supérieure (28), une portion au moins dudit élément de pontage (40) étant disposée en relation de chevauchement avec une section d'extrémité longitudinale dudit corps absorbant (32) et connectée à l'une au moins de ladite couche de feuille support (30) et de ladite couche de feuille supérieure (28) ;
des panneaux latéraux élastiqués (56,58) construits pour être élastiquement extensibles le long d'au moins une direction transversale dudit article (10) ; et
un moyen d'attache (36) pour fixer ledit article (10) sur un porteur, ledit moyen d'attache (36) étant connecté à une région d'extrémité transversalement distale de l'un au moins desdits panneaux latéraux (56,58) ;
ledit article étant caractérisé en ce que les panneaux latéraux élastiqués (56,58) sont fournis séparés dudit élément de pontage et sont connectés audit élément de pontage (40) au niveau de chacune des sections d'extrémité transversalement opposées (84,86) dudit élément de pontage (40) ; en ce que ledit élément de pontage (40) s'étend transversalement pour interconnecter lesdits panneaux latéraux élastiqués (56,58) ; et en ce que ledit élément de pontage (40) est configuré pour former un sous-ensemble avec lesdits panneaux latéraux, ledit sous-ensemble maintenant une séparation transversale et un alignement voulus entre lesdits panneaux latéraux (56,58) fournis séparément, lorsque ledit sous-ensemble est positionné dans ledit article au cours de la fabrication.

2. Article absorbant (10) selon la revendication 1, dans lequel ledit élément de pontage (40) a une dimension dans le sens longitudinal qui est inférieure à la longueur de la feuille support et inférieure à la longueur de ladite feuille supérieure.

3. Article absorbant (10) selon la revendication 2, dans lequel ledit élément de pontage (40) est connecté entre ladite couche de feuille support (30) et ladite couche de feuille supérieure (28) et est disposé de façon à chevaucher une surface adjacente côté corporel, intérieure, dudit corps absorbant 32.

4. Article absorbant (10) selon la revendication 3, dans lequel ledit élément de pontage (40) a une région de bord fixe s'étendant transversalement (96) et une paire de régions latérales fixes s'étendant longitudinalement (102).

5. Article absorbant (10) selon la revendication 4, dans lequel ledit élément de pontage (40) est construit pour empêcher sensiblement un passage de liquide au travers de son épaisseur.

6. Article absorbant (10) selon la revendication 5, dans lequel ledit élément de pontage (40) est perméable à une traversée de gaz.

7. Article absorbant (10) selon la revendication 2, dans lequel ledit élément de pontage (40) est connecté en une relation de vis-à-vis adjacente avec une surface côté corporel principale de ladite couche de feuille supérieure (28).

8. Article absorbant (10) selon la revendication 7, dans lequel ledit élément de pontage (40) est construit pour empêcher sensiblement un passage de liquide au travers de son épaisseur.

9. Article absorbant (10) selon la revendication 8, comprenant en outre une paire de volets de retenue s'étendant longitudinalement, dans lequel ledit élément de pontage (40) est interposé entre lesdits volets de retenue (62) et ladite couche de feuille supérieure (28).

10. Article absorbant (10) selon la revendication 9, dans lequel ledit élément de pontage (40) a une région de bord fixe s'étendant transversalement (96) et une paire de régions latérales fixes (102) s'étendant longitudinalement et transversalement opposées.

11. Article absorbant (10) selon la revendication 10, dans lequel ladite région de bord fixe s'étendant transversalement (96) et lesdites régions latérales fixes s'étendant longitudinalement (102) dudit élément de pontage sont fixées pour réaliser des joints opérationnels qui peuvent empêcher sensiblement toute fuite indésirable de liquide au-delà desdites régions de bord fixe.

12. Article absorbant (10) selon la revendication 2, dans lequel ledit article comprend en outre une paire de volets de retenue s'étendant longitudinalement (62) et ledit élément de pontage (40) est connecté de façon à ce que sa portée aille d'un desdits volets de retenue (62) à l'autre et pour offrir une surface côté corporel destinée à venir en contact avec le porteur.

13. Article absorbant (10) selon la revendication 12, dans lequel ledit élément de pontage (40) a un région de bord fixe s'étendant transversalement et une paire de régions latérales fixes s'étendant longitudinalement et transversalement opposées.

14. Article absorbant (10) selon la revendication 13, dans lequel ledit élément de pontage (40) est construit pour empêcher sensiblement un passage de liquide au travers de son épaisseur.

15. Article absorbant (10) selon la revendication 14, dans lequel ledit élément de pontage (40) est perméable aux gaz.

16. Article absorbant (10) selon la revendication 15, dans lequel ledit moyen d'attache (36) comprend une première attache connectée à une région d'extrémité transversalement distale d'un premier panneau latéral (56) et une seconde attache connectée à une région d'extrémité transversalement distale d'un second panneau latéral (58).

17. Article absorbant (10) selon la revendication 15, dans lequel lesdites première et seconde attaches comprennent des régions de liaison utilisateur adhésives.

18. Article absorbant (10) selon la revendication 15, dans lequel lesdites première et seconde attaches comprennent des régions de liaison utilisateur mécaniques.
